(19) Europäisches Patentamt
European Patent Office
Office européen des brevets

(11) EP 0 687 664 B1

(12) **FASCICULE DE BREVET EUROPEEN**

(45) Date de publication et mention
de la délivrance du brevet:
**22.10.1997 Bulletin 1997/43**

(51) Int. Cl.$^6$: **C07C 67/08**, C07C 69/54

(21) Numéro de dépôt: **95401428.8**

(22) Date de dépôt: **16.06.1995**

(54) **Procédé de fabrication d'acrylates d'alkyle par estérification directe**

Verfahren zur Herstellung von Alkylacrylaten durch direkte Veresterung

Process for the production of alkyle acrylates by direct esterification

(84) Etats contractants désignés:
**AT BE CH DE DK ES FR GB GR IE IT LI LU NL PT SE**

(30) Priorité: **17.06.1994 FR 9407453**

(43) Date de publication de la demande:
**20.12.1995 Bulletin 1995/51**

(73) Titulaire: **ELF ATOCHEM S.A.**
**92800 Puteaux (FR)**

(72) Inventeurs:
• **Bessalem, Jacqueline**
**F-57500 Saint-Avold (FR)**
• **Fauconet, Michel**
**F-57730 Valmont (FR)**
• **Lacroix, Christian**
**F-57600 Folkling (FR)**
• **Hess, Nathalie**
**F-57500 Saint-Avold (FR)**

(74) Mandataire: **Chaillot, Geneviève**
**Cabinet CHAILLOT,**
**16-20, avenue de L'Agent Sarre,**
**B.P. 74**
**92703 Colombes Cédex (FR)**

(56) Documents cités:
**US-A- 4 280 009**     **US-A- 4 833 267**

**Description**

La présente invention porte sur un perfectionnement au procédé de fabrication d'acrylates d'alkyle par estérification directe.

Dans les procédés d'estérification, il est nécessaire, pour atteindre une conversion maximale de l'alcool et de l'acide, de déplacer vers la droite l'équilibre réactionnel :

$$R\text{-}COOH + R'\text{-}OH \Leftrightarrow R\text{-}COOR' + H_2O \ .$$

Dans cet objectif, le procédé le plus communément utilisé consiste à distiller le produit le plus léger, en l'occurrence l'eau, au fur et à mesure de sa formation. Pour optimiser la cinétique de réaction, il est important de limiter au minimum l'eau présente dans le milieu réactionnel, en l'extrayant le plus rapidement possible, afin d'éviter toute réaction inverse.

En particulier, dans le domaine de la synthèse des esters acryliques, qui sont réputés thermiquement instables (polymérisation favorisée par effet thermique), il est classique et énergétiquement moins coûteux de distiller l'eau sous forme d'un azéotrope à point d'ébullition inférieur.

L'azéotrope peut être un homo- ou un hétéroazéotrope, formant respectivement un système mono- ou biphasique avec l'eau. Dans le cas de la distillation d'un homoazéotrope, la sélectivité de la colonne de distillation ne peut être assurée qu'au prix du reflux d'une partie de ce mélange azéotrope en tête de colonne. Ceci entraîne un retour d'eau dans le milieu réactionnel, qui diminue la cinétique de la réaction d'estérification.

Au contraire, si le mélange est un hétéroazéotrope, celui-ci peut être séparé en une phase organique supérieure et une phase aqueuse inférieure dans un décanteur situé en tête de colonne à distiller, et le reflux nécessaire pour assurer une bonne sélectivité dans la colonne peut être obtenu par recyclage de la seule phase organique. Dans ce cas, l'eau est habituellement soutirée de manière continue en fond de décanteur.

Le corps utilisé pour entraîner azéotropiquement l'eau de réaction est généralement un solvant non miscible et formant un azéotrope avec l'eau, comme il est décrit dans les brevets américains US-A-5 093 520 et US-A-2 917 538 ; cependant, ce procédé présente le désavantage d'ajouter un nouveau corps dans le mélange réactionnel, qui devra être séparé par la suite et éventuellement purifié avant recyclage en amont dans le procédé.

Il est également décrit d'utiliser l'alcool estérifiant, particulièrement s'il est non miscible avec l'eau, comme solvant azéotropeur, ce qui évite d'utiliser un nouveau corps dans le procédé.

Dans le cas de la fabrication d'esters acryliques d'alcools ayant un nombre d'atomes de carbone supérieur ou égal à 5, comme l'acrylate de 2-éthylhexyle, décrit dans le brevet américain US-A-4 280 009, ce procédé ne peut être utilisé simplement. L'hétéroazéotrope formé par le mélange ester-alcool-eau possède une température d'ébullition élevée et relativement proche de celle de l'acide acrylique. De ce fait, une partie de l'acide acrylique distille en même temps que l'eau et l'alcool entraîneur. De façon générale, pour l'estérification d'alcools présentant un point d'ébullition plus élevé que celui de l'acide acrylique, le composé le plus léger (acide acrylique) est entraîné dans les vapeurs, surtout lorsqu'en fin de réaction, la quantité d'eau à extraire du milieu réactionnel, nécessaire à la constitution du mélange azéotrope léger, devient très faible.

L'entraînement d'acide acrylique devient ainsi particulièrement important lorsqu'on vise un taux de transformation total des réactifs.

Après séparation, une partie importante de cet acide acrylique se retrouve dans la phase aqueuse, ce qui occasionne une perte de produit et une augmentation de la pollution organique (DCO) des eaux rejetées par l'atelier.

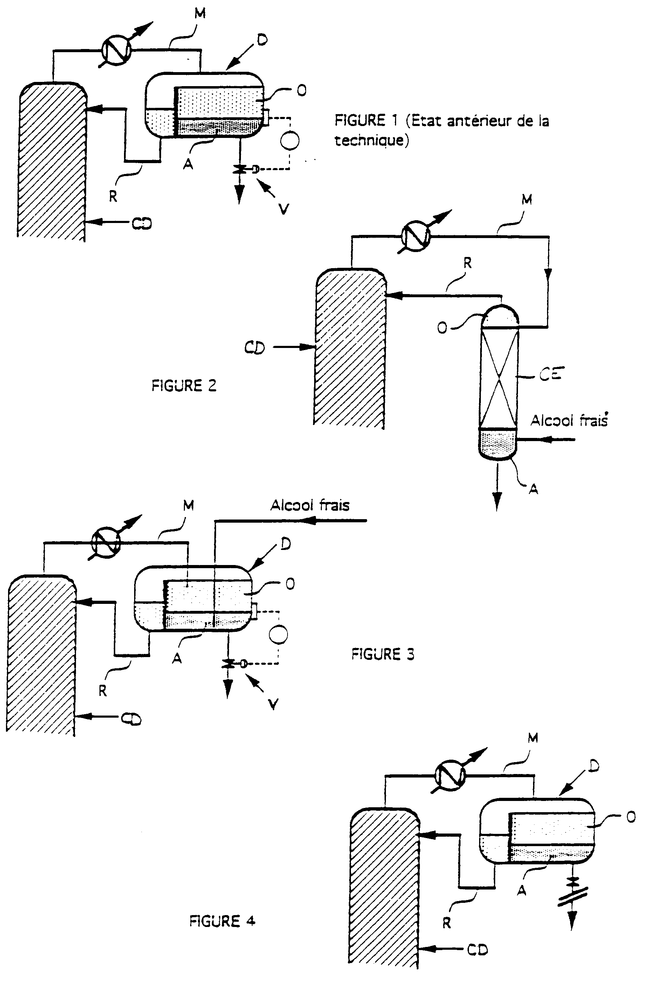

Si l'on se réfère à la Figure 1 du dessin annexé, on peut voir que, dans une estérification réalisée selon la technique habituelle, les vapeurs condensées en tête de la colonne de distillation (CD) pendant la réaction (mélange hétéroazéotropique M) sont collectées dans un décanteur (D) où le mélange se sépare en deux phases. La phase organique supérieure (O) est renvoyée par débordement (R) (reflux naturel) en tête de la colonne (CD). La phase aqueuse (A) est, quant à elle, soutirée de manière continue par l'intermédiaire d'une vanne (V), régulant un niveau d'interphase constant dans le décanteur (D).

Lorsqu'on applique ce procédé à la synthèse d'acrylates lourds, la teneur en acide acrylique dans la phase aqueuse récupérée après réaction est très importante.

Pour diminuer l'entraînement d'acide acrylique dans l'azéotrope distillé pendant la réaction, on peut augmenter le taux de reflux à l'intérieur de la colonne par introduction d'une partie de l'alcool estérifiant en tête de celle-ci, comme il est décrit dans les brevets américains US-A-4 833 267 et US-A-4 280 009, dans le brevet roumain RO-70 951 et dans la demande de brevet japonais JP-A-58 192 851.

Cette méthode nécessite d'ajouter, par la tête de la colonne, une proportion importante d'alcool par rapport à la totalité de l'alcool nécessaire à la réaction, et la technique est peu efficace pour éviter la distillation de l'acide acrylique dans le cas d'une synthèse de l'acrylate de 2-éthylhexyle, particulièrement lorsque l'on vise une conversion totale des réactifs.

Par ailleurs, dans le brevet américain US-A-4 076 950, est décrit un procédé d'estérification visant à distiller, pendant la réaction, un mélange azéotropique exempt d'acide acrylique. Pour ce faire, on reflue en tête de colonne la phase organique préalablement débarrassée de l'alcool de départ par une extraction à l'eau, et qui contient alors essentiellement de l'ester anhydre. Cette procédure n'est pas adaptable dans le cas d'estérification d'alcools lourds (alcools en $C_n$ avec $n \geq 5$), du fait des très faibles solubilités des alcools dans l'eau.

Le problème que la Société déposante a cherché à résoudre, dans la synthèse des acrylates fabriqués à partir d'alcools ayant un nombre d'atomes de carbone supérieur ou égal à 5, par estérification directe, réalisée de façon continue ou discontinue en l'absence de solvant azéotropeur externe, est celui de réduire au maximum la teneur en acide acrylique dans les eaux épuisées rejetées par l'atelier, afin de diminuer la perte de ce monomère et la pollution organique (DCO) qu'il engendre.

A cet effet, il est prévu, conformément à la présente invention, de renvoyer en tête de la colonne de distillation surmontant le réacteur, l'acide acrylique entraîné pendant la distillation du mélange hétéroazéotropique, après extraction de cet acide contenu dans l'eau de réaction par un mélange riche en alcool estérifiant.

La présente invention a donc pour objet un procédé de fabrication d'un ester d'acide acrylique par réaction dudit acide et d'un alcool choisi parmi les monoalcools primaires et secondaires aliphatiques ayant un nombre d'atomes de carbone supérieur ou égal à 5, en présence d'un acide comme catalyseur d'estérification et d'un inhibiteur de polymérisation, l'alcool étant capable de former un hétéroazéotrope avec l'eau, l'eau formée par la réaction d'estérification étant entraînée par distillation dans une colonne sous la forme d'un mélange hétéroazéotropique avec l'alcool, lequel mélange, comportant une quantité d'acide acrylique n'ayant pas réagi, est soumis ensuite, après condensation, à une séparation pour donner une phase organique supérieure que l'on recycle en tête de la colonne de distillation et une phase aqueuse inférieure que l'on soutire, caractérisé par le fait que l'on conduit une extraction de l'acide acrylique contenu dans le mélange hétéroazéotropique condensé adressé à la séparation de phases par un mélange riche en alcool estérifiant, et qu'on renvoie en tête de la colonne de distillation, l'acide acrylique ainsi extrait, en tant que constituant de la phase organique recyclée, avec augmentation du taux de reflux dans la colonne de distillation par rapport au reflux naturel.

Divers moyens permettent cette augmentation du taux de reflux dans la colonne de distillation, ces moyens pouvant notamment dépendre du fait que l'on travaille en continu ou en discontinu.

Conformément à un premier mode de réalisation du procédé selon l'invention, illustré de façon schématique sur la Figure 2, l'estérification est conduite en continu, et l'on effectue l'opération d'extraction dans une colonne d'extraction (CE) alimentée en tête, par le mélange hétéroazéotropique condensé (M), et en pied, par de l'alcool frais.

Conformément à un second mode de réalisation du procédé de l'invention, illustré de façon schématique sur la Figure 3, l'estérification est conduite en continu, et l'on effectue l'opération d'extraction dans un décanteur (D) alimenté, dans la partie supérieure de sa phase organique surnageante (O), par le mélange hétéroazéotropique condensé (M), et, dans la partie inférieure de sa phase aqueuse inférieure (A), par de l'alcool frais, et l'on procède de façon à soutirer à niveau d'interphase constant dans le décanteur (D).

Conformément à un troisième mode de réalisation du procédé de l'invention, illustré de façon schématique également sur la Figure 3, l'estérification est conduite en discontinu, en mettant en oeuvre le procédé décrit dans le paragraphe précédent.

Conformément à un quatrième mode de réalisation du procédé de l'invention, illustré de façon schématique sur la Figure 4, l'estérification est conduite en discontinu, et l'on effectue l'opération d'extraction dans un décanteur (D) dimensionné de façon à pouvoir recevoir au moins la totalité du mélange hétéroazéotropique (M) sans soutirage au fond dudit décanteur (D) ; avant le démarrage de la réaction, on remplit le décanteur (D) d'alcool estérifiant jusqu'au niveau de débordement dans la colonne de distillation (CD) ; on amène le mélange hétéroazéotropique condensé (M), que l'on fait arriver dans la partie supérieure de la phase organique (O) qui se forme dans le décanteur (D), à s'accumuler dans ce dernier pendant tout ou partie de la réaction, le volume de phase organique supérieure (O) déplacé par le volume de mélange hétéroazéotropique accumulé étant reflué (par R) en tête de la colonne de distillation (CD) ; et lorsque la réaction est terminée, on soutire la phase aqueuse inférieure (A) et on remplace le volume du mélange hétéroazéotropique éliminé par de l'alcool frais pour une prochaine réaction d'estérification.

Conformément à un cinquième mode de réalisation du procédé de l'invention, l'estérification est conduite en discontinu, en mettant en oeuvre le procédé décrit dans le paragraphe précédent, la seule différence étant qu'on introduit de l'alcool frais dans le décanteur pendant tout ou partie de la réaction.

Le mélange hétéroazéotropique alcool/eau présente une composition très riche en eau (90% en volume) et pauvre en alcool (10%). De ce fait, en soutirant l'eau avec une régulation du niveau de l'interphase dans le décanteur, le volume reflué dans la colonne représente 10% du liquide issu des vapeurs condensées. Conformément à l'invention, en augmentant ce reflux naturel par introduction continue d'alcool frais dans le décanteur (procédé continu ou discontinu) et/ou en accumulant l'eau sans soutirage en pied de décanteur pendant toute la réaction (procédé discontinu), on augmente le taux de reflux dans la colonne de distillation. Dans le cas du procédé selon l'invention d'estérification discontinue avec soutirage différé de l'eau de réaction, on reflue en tête de colonne au moins la totalité (100%) du volume de liquide (phase organique + phase aqueuse) condensé (soutirage différé sans introduction continue d'alcool frais). Ceci revient donc à multiplier par un facteur au moins égal à 10 la quantité de liquide reflué dans la colonne, donc à améliorer sa sélectivité.

Dans le procédé selon l'invention, le décanteur ou la colonne d'extraction liquide-liquide permettent de réextraire l'acide acrylique entraîné dans l'eau de réaction. Les vapeurs issues de la colonne sont condensées et le liquide se sépare en deux phases. La phase aqueuse traverse la masse d'alcool frais présent dans le décanteur avant réaction ou dans la phase supérieure de la phase organique de la colonne, et grâce à son coefficient de partage favorable à la phase alcoolique, une partie importante de l'acide acrylique contenu dans cette phase aqueuse passe dans la phase organique. En outre, l'addition continue d'alcool dans la phase aqueuse inférieure du décanteur (procédé continu ou discontinu) ou de la colonne d'extraction (procédé continu) permet d'extraire à nouveau l'acide acrylique dans cette phase aqueuse.

La phase aqueuse (E) obtenue dans le procédé de l'invention, est ensuite envoyée, avantageusement avec les autres eaux de l'atelier (eaux de neutralisation du brut réactionnel, eaux de lavage du brut neutralisé, eaux de piégeage des évents gazeux, ...), dans une colonne de distillation en aval permettant de récupérer les produits organiques légers en tête et de rejeter les eaux épuisées en pied. La DCO représentative de l'acide acrylique entraîné pendant la réaction est alors nettement réduite par rapport à une conduite à niveau d'interphase constant sans rajout d'alcool frais dans le décanteur.

Conformément à la présente invention, on peut également avantageusement conduire dans la colonne de lavage une extraction des dernières traces d'acide acrylique contenues dans la phase aqueuse issue de l'opération de séparation, par le brut réactionnel débarrassé du catalyseur et de tout ou partie de l'acide acrylique par neutralisation.

Cette amélioration présente plusieurs avantages :

- elle permet de réduire la pollution organique, sous forme de DCO due à l'acide acrylique, rejetée en pied de colonne de distillation des eaux de l'atelier ;
- pour des esters dont le point d'ébullition est supérieur à celui de l'acide acrylique, comme l'acrylate de 2-éthylhexyle, elle permet de récupérer l'acide acrylique résiduel entraîné dans l'eau de réaction, dans les légers de la colonne d'étêtage, pour recyclage à l'étape réactionnelle ;
- en se substituant à une addition d'eau pure, elle diminue la consommation d'eau de l'atelier, et par conséquent la quantité d'eau rejetée à traiter.

La réaction d'estérification est généralement conduite à une température de 80 à 130°C, à pression normale ou sous une pression réduite. Comme exemple d'alcool, on peut citer le 2-éthylhexanol.

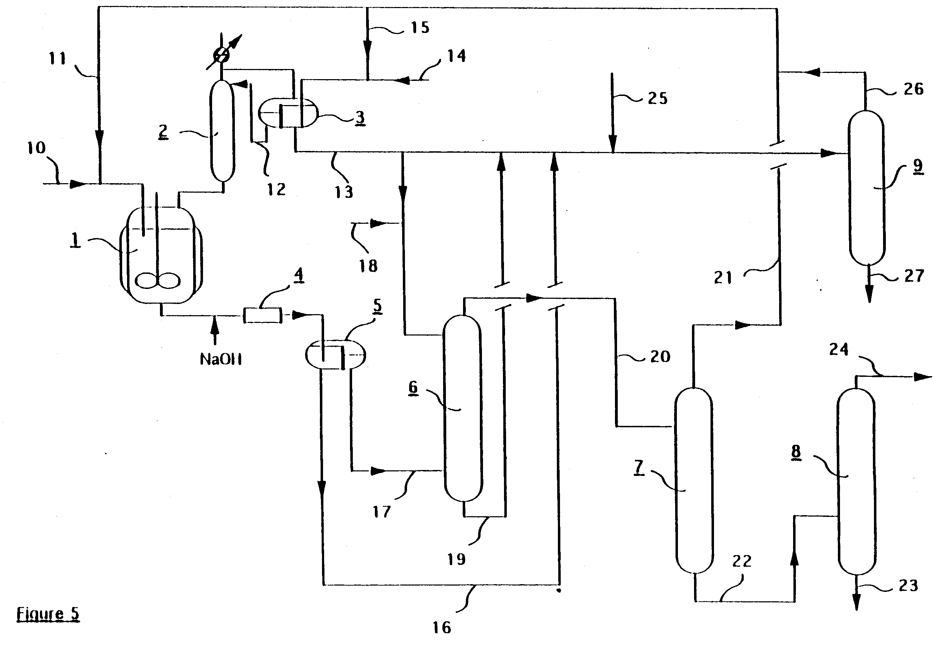

Sur le dessin annexé, en dehors des Figures 1 à 4 déjà décrites, on a représenté, sur une Figure 5, un schéma d'un mode de réalisation particulier du procédé selon l'invention, que l'on va maintenant décrire.

Dans le réacteur 1 surmonté d'une colonne de distillation 2, on introduit (en discontinu ou en continu) l'acide acrylique frais, l'alcool frais, les stabilisants et le catalyseur ($H_2SO_4$) par la ligne 10, ainsi que, par la ligne 11, le flux de composés légers (essentiellement alcool et ester) récupérés dans les colonnes d'étêtage ultérieures. La température du milieu réactionnel est comprise entre 80 et 130°C, préférentiellement entre 80 et 100°C, sous une pression réduite de façon à maintenir la température désirée. Pendant la réaction, on distille un mélange d'alcool, d'ester, d'eau et d'acide acrylique, qui se sépare, après condensation, en deux phases dans le décanteur 3. La phase organique 12 est renvoyée par débordement en tête de colonne 2, tandis que la phase aqueuse 13 est envoyée à l'alimentation de la colonne de distillation 9 et/ou en tête de la colonne de lavage 6. Pendant la réaction, on introduit dans le décanteur 3, par la conduite 14, un flux d'alcool frais et/ou de composés légers 15 récupérés dans les colonnes d'étêtage.

Après réaction, le brut réactionnel est neutralisé par une solution aqueuse sodique dans le mélangeur 4 pour éliminer le catalyseur et/ou l'acide acrylique résiduel. Le mélange hétérogène est séparé dans le décanteur 5 en une phase aqueuse 16 qui est envoyée à l'alimentation de la colonne 9 et une phase organique qui constitue l'alimentation en pied de la colonne de lavage 6.

Cette colonne de lavage 6 est, par ailleurs, alimentée en tête par de l'eau déminéralisée 18 et/ou tout ou partie de l'eau de réaction 13. En pied de colonne 6, on récupère un flux aqueux alcalin 19, qui rejoint l'alimentation de la colonne 9.

En tête de colonne 6, le brut neutralisé et lavé 20 est envoyé à la colonne d'étêtage 7, qui permet de récupérer en tête l'alcool excédentaire et l'acide acrylique extrait de l'eau de réaction, ainsi qu'une partie d'ester. Ce mélange 21 est recyclé à l'étape réactionnelle via la ligne 11.

Le brut étêté récupéré 22 en pied de la colonne 7 alimente la colonne d'équeutage 8, qui élimine en pied les lourds 23, pour obtenir en tête l'ester pur 24.

Toutes les phases aqueuses de l'atelier : eau de réaction 13, eau de neutralisation du brut 16, eau de la colonne de lavage 19, autres eaux de l'atelier 25, sont envoyées sur le colonne 9, en tête (26) de laquelle on récupère principalement l'alcool, qui est recyclé à l'étape réactionnelle via la conduite 11. Les eaux épuisées 27, débarrassées de l'essentiel des produits organiques polluants, sont éliminées pour traitement biologique ultérieur avant rejet.

La présente invention va maintenant être encore illustrée par des exemples donnés à titre illustratif et non limitatif. Dans ces Exemples, les pourcentages sont indiqués en poids sauf indication contraire et les abréviations suivantes ont été utilisées :

AA = acide acrylique
2EHOH = 2-éthylhexanol
A2EH = acrylate de 2-éthylhexyle.

EXEMPLES 1 à 5

On opère de façon discontinue dans un équipement constitué par :

- un réacteur chauffé d'une capacité de 150 litres, équipé d'un agitateur mécanique, d'une sonde de température, d'une arrivée d'air pour assurer un léger bullage dans le milieu réactionnel ; ce réacteur est surmonté d'une colonne adiabatique d'efficacité 4 plateaux théoriques;
- un conduit permettant d'envoyer éventuellement une partie de l'alcool estérifiant et/ou de l'ester à recycler en tête de la colonne ;
- un condenseur alimenté par un mélange d'eau glycollée maintenu à une température de 0°C ;
- une sonde de mesure de température en tête de colonne ;
- un décanteur recueillant l'azéotrope condensé, muni d'un système permettant éventuellement de maintenir un niveau constant de phase aqueuse inférieure par soutirage automatique de l'eau de réaction au fur et à mesure de sa formation grâce à l'ouverture d'une électrovanne. La phase organique supérieure est renvoyée en tête de colonne par débordement naturel. Le conduit d'arrivée du liquide condensé arrive dans la partie supérieure de la phase légère, et un deuxième conduit permet d'envoyer éventuellement une partie de l'alcool estérifiant et/ou de l'ester à recycler dans la partie inférieure de la phase aqueuse lourde ; et
- un système de vide régulé permettant de travailler sous pression réduite.

Dans le réacteur, on charge l'acide acrylique ; l'alcool, en quantité telle que le rapport molaire de ce composé sur l'acide acrylique initial soit égal à 1,1 (pour l'Exemple 5, une partie de l'alcool prévu pour la réaction est introduite de façon continue dans le décanteur, pendant tout ou partie de la réaction) ; pour les Exemples 3 à 5, une quantité d'ester A2EH variant entre 15 et 20% par rapport à la charge totale, pour simuler le recyclage du mélange ester-alcool récupéré en tête de la colonne 7 (flux 21) ; le stabilisant phénothiazine, ajouté à raison de 0,06% par rapport à la charge totale ; le catalyseur acide sulfurique en solution à 95%, à raison de 1% par rapport à la charge totale.

Dans le décanteur de réaction 3, on introduit de l'alcool 2EHOH jusqu'à la limite du débordement vers la tête de colonne 2 (sauf pour l'Exemple 1).

Lorsque tous les réactifs ont été introduits dans le réacteur, on porte le mélange à 85°C (Exemples 1 à 3) ou à 85-100°C (Exemples 4 et 5) sous $3,3 \times 10^4$ Pa (250 mmHg) et on régule la température désirée en appliquant la pression nécessaire pour maintenir cette température.

La réaction est considérée comme terminée lorsque la teneur en acide acrylique résiduel atteint 0,5%. Ramené sous pression atmosphérique, le brut est refroidi à 40°C et neutralisé par une solution aqueuse de soude à 8% à raison d'un rapport molaire NaOH/acidité totale de 1,05.

EXEMPLE 1 (comparatif)

On procède au soutirage total du mélange hétéroazéotropique, sans aucun reflux. On observe une très forte teneur en AA dans la phase aqueuse.

EXEMPLE 2 (comparatif)

On procède à un soutirage à niveau d'interphase constante. On observe une très forte teneur en AA dans l'eau de réaction.

EXEMPLE 3

On procède à un soutirage différé. On observe une faible teneur en AA dans l'eau de réaction.

EXEMPLE 4

On procède comme à l'Exemple 3, mais à température plus élevée, ce qui permet d'obtenir un temps de réaction plus court en conservant un faible passage d'acide acrylique dans l'eau de réaction.

EXEMPLE 5

On procède à un soutirage à niveau d'interphase constant, avec introduction en continu dans le décanteur d'une partie de l'alcool prévu pour la réaction. Ce mode opératoire donne aussi de bons résultats en teneur en AA dans l'eau de réaction.

Cette façon de procéder pendant la réaction est compatible avec une synthèse en continu de l'A2EH.

Les résultats de ces Exemples 1 à 5 sont présentés dans le Tableau 1 suivant.

Tableau 1

| Exemple | 1 (comparatif) | 2 (comparatif) | 3 | 4 | 5 |
|---|---|---|---|---|---|
| Ester recyclé/ charge (%) | 0 | 0 | 15 | 18 | 18 |
| Température de réaction (°C) | 85 | 85 | 85 | 85-95 | 85-100 |
| Mode de soutirage de la phase aqueuse | Soutirage total, sans reflux | Soutirage à niveau d'interphase constant* | Soutirage différé | Soutirage différé | Soutirage continu, avec rajout continu d'alcool dans le décanteur |
| Contenu initial du décanteur | nul | 2EHOH | 2EHOH | 2EHOH | 2EHOH |
| Volume du mélange azéotropique condensé (litres) | 9 | 9 | 9 | 9 | 9 |
| Volume de la phase organique refluée (litres) | | | | | |
| - reflux naturel du décanteur | 0 | 0,9 | 9 | 9 | 0,9 |
| - ajout 2EHOH dans le décanteur** | 0 | 0 | 0 | 0 | 2,4 |
| Rapport reflux/mélange hétéroazéotropique condensé (v/v) | 0 | 0,1/1 | 1/1 | 1/1 | 2,8/1 |
| Temps de réaction (mn) | 240 | 270 | 390 | 300 | 300 |
| AA dans le brut réactionnel (%) | 0,45 | 0,55 | 0,51 | 0,37 | 0,51 |
| AA dans l'eau de réaction | | | | | |
| - AA/phase aqueuse (%) | 12,7 | 14,5 | 1,8 | 0,7 | 2,2 |
| - AA phase aqueuse/AA initial (%) | 3,5 | 4,1 | 0,4 | 0,19 | 0,46 |

* sans ajout d'alcool

** pas d'ajout de 2EHOH en tête de la colonne de distillation

EXEMPLES 6 à 14

On procède comme pour les Exemples 1 à 5, mais dans un réacteur chauffé d'une capacité de 1 litre. Pour la réalisation de certains exemples, une partie de l'alcool prévu pour la réaction est introduite de façon continue dans le décanteur ou en tête de colonne, pendant tout ou partie de la réaction.

EXEMPLE 6

On conduit la réaction à une température de 85°C. On procède à un soutirage différé sans recyclage d'A2EH. On observe une faible teneur en AA dans l'eau de réaction et un bon temps de réaction.

EXEMPLE 7 (comparatif)

On procède comme à l'Exemple 6, mais le reflux est assuré par introduction d'alcool en tête de colonne et non dans le décanteur, selon un débit en fonction du temps de réaction exactement identique à celui du liquide condensé en tête de colonne, donc simulant parfaitement un reflux naturel via le décanteur. Les résultats sont nettement moins bons et l'on observe un entraînement important d'AA dans l'eau de réaction.

On montre ainsi que les deux effets (augmentation de la quantité de liquide reflué dans la colonne et extraction d'acide acrylique par l'alcool frais présent dans le décanteur) sont complémentaires et nécessaires pour réduire la teneur en acide acrylique dans l'eau de réaction.

EXEMPLE 8

On procède comme à l'Exemple 6, mais avec recyclage d'A2EH. Les résultats sont équivalents à ceux de l'Exemple 6.

EXEMPLE 9

On procède comme à l'Exemple 8, mais en partant d'un décanteur rempli de mélange A2EH/2EHOH 80/20 en volume, au lieu d'un décanteur rempli de 2EHOH. On observe un peu plus d'entraînement d'AA dans l'eau de réaction, qui s'explique par un taux d'extraction d'AA dans la phase organique plus favorable dans le cas de l'alcool que dans le cas de l'ester.

EXEMPLE 10

On procède comme à l'Exemple 8, mais à la température de réaction de 85-100°C, avec soutirage différé de l'eau de réaction. On observe peu d'entraînement d'AA, un temps de réaction fortement diminué et une productivité augmentée.

EXEMPLE 11 (comparatif)

On procède comme à l'Exemple 10, mais avec soutirage du mélange azéotropique à niveau d'interphase constant et envoi du complément d'alcool en continu, en tête de colonne, pour assurer un même taux de reflux. Le fait de ne pas passer par le décanteur dégrade fortement l'entraînement d'AA dans l'eau de réaction.

EXEMPLE 12 (comparatif)

On procède comme à l'Exemple 10, mais avec soutirage total de l'azéotrope condensé et envoi du complément d'alcool en continu, en tête de colonne, pour assurer un même taux de reflux. On formule les mêmes observations qu'à l'Exemple 11.

EXEMPLE 13

On procède comme à l'Exemple 10, avec soutirage différé de l'eau de réaction, mais avec, en plus, une addition supplémentaire d'alcool frais, en continu, dans le décanteur. On observe de très bons résultats en entraînement d'AA dans l'eau de réaction, améliorés par le renouvellement continu de l'alcool frais.

EXEMPLE 14

On procède comme à l'Exemple 12, mais avec un soutirage à niveau constant de l'eau de réaction. On obtient des résultats équivalents.

Tableau 2

| Exemple | 6 | 7 (comparatif) | 8 | 9 | 10 | 11 (comparatif) | 12 (comparatif) | 13 | 14 |
|---|---|---|---|---|---|---|---|---|---|
| Ester recyclé/charge (%) | 0 | 0 | 15 | 15 | 20 | 20 | 20 | 20 | 20 |
| Température de réaction (°C) | 85 | 85 | 85 | 85 | 85-100 | 85-100 | 85-100 | 85-100 | 85-100 |
| Mode de soutirage de la phase aqueuse | Soutirage différé | Soutirage total, avec reflux par introduction de 2EHOH en tête de colonne | Soutirage différé | Soutirage différé | Soutirage différé | Soutirage à niveau d'interphase constant, avec rajout continu d'alcool en tête de colonne | Soutirage total, avec reflux par introduction de 2EHOH en tête de colonne | Soutirage différé, avec rajout continu d'alcool dans le décanteur | Soutirage continu, avec rajout continu d'alcool dans le décanteur |
| Contenu initial du décanteur | 2EHOH | 2EHOH | 2EHOH | 2EHOH/A2EH 20/80 | 2EHOH | 2EHOH | 2EHOH | 2EHOH | 2EHOH |
| Volume du mélange azéotropique condensé (ml) | 73,5 | 73,5 | 73,5 | 73,5 | 73,5 | 73,5 | 73,5 | 73,5 | 73,5 |
| Volume de la phase organique refluée (ml) |  |  |  |  |  |  |  |  |  |
| - reflux naturel du décanteur | 73,4 | 0 | 73,4 | 73,4 | 73,4 | 8,7 | 0 | 73,4 | 8,7 |
| - ajout 2EHOH dans le décanteur | 0 | 0 | 0 | 0 | 0 | 0 | 0 | 129,4 | 194,2 |
| - ajout 2EHOH en tête de colonne | 0 | 64,7 | 0 | 0 | 0 | 64,7 | 71,9 | 0 | 0 |
| Rapport reflux/mélange azéotropique condensé (v/v) | 1/1 | 0,9/1 | 1/1 | 1/1 | 1/1 | 1/1 | 1/1 | 2,8/1 | 2,8/1 |
| Temps de réaction (mn) | 290 | 315 | 270 | 290 | 195 | 210 | 190 | 210 | 195 |
| AA dans le brut réactionnel (%) | 0,48 | 0,36 | 0,47 | 0,41 | 0,32 | 0,4 | 0,38 | 0,35 | 0,33 |
| AA dans l'eau de réaction |  |  |  |  |  |  |  |  |  |
| - AA/phase aqueuse (%) | 1,1 | 5,7 | 1,4 | 3,5 | 2,1 | 5,3 | 6,2 | 0,8 | 1,2 |
| - AA phase aqueuse/AA initial (%) | 0,27 | 1,35 | 0,34 | 0,88 | 0,52 | 1,22 | 1,65 | 0,2 | 0,3 |
| Rendement A2EH/AA (%) | 97,8 | 93,9 | 96,8 | 97,5 | 98,3 | 95 | 96,3 | 98,9 | 97 |

EXEMPLES 15 et 16

Pour démontrer l'efficacité d'une réextraction, dans la colonne de lavage 6, de l'AA résiduel entraîné dans la phase aqueuse réactionnelle par le mélange réactionnel brut neutralisé, en utilisant comme eau de lavage cette phase aqueuse, on a simulé exactement une colonne industrielle en utilisant une batterie de laboratoire de trois mélangeur-décanteurs, chaque étage de mélangeur-décanteur représentant un étage théorique d'une colonne de lavage industrielle et réalisant les opérations suivantes : mise en contact intime dans un compartiment mélangeur, grâce à une turbine, de la phase aqueuse obtenue pendant la réaction d'estérification et de la phase organique constituée par le mélange brut réactionnel neutralisé ; et séparation en deux phases, par gravité, de l'émulsion formée, dans un compartiment décanteur associé à chaque mélangeur. Les deux phases passent ensuite, à contre-courant, dans les étages contigus.

EXEMPLE 15

A partir d'une réaction d'estérification d'AA par 2EHOH réalisée au laboratoire dans les conditions optimales décrites ci-dessus, on récupère un mélange brut de composition suivante :

- A2EH : 91% ;
- 2EHOH : 4% ;
- AA : 0,5% ;
- Sulfate acide de 2-éthylhexyle : 2,7%.

Ce mélange est neutralisé par une solution aqueuse de soude à 8%, à raison d'un rapport molaire de soude à la totalité des espèces acides de 1,05. Après séparation des deux phases obtenues, on vérifie que la phase organique est exempte d'AA et de sulfate de 2-éthylhexyle.

La même réaction permet de récupérer une phase aqueuse dans le décanteur situé en tête de la colonne de réaction, dont l'analyse est la suivante :

- $H_2O$ : 98,2% ;
- 2EHOH : 0,09% ;
- AA : 1,7%.

La phase organique constituée par le brut réactionnel neutralisé est envoyée dans le compartiment mélangeur du premier étage d'extraction liquide-liquide, tandis que la phase aqueuse réactionnelle est introduite dans le compartiment du troisième étage d'extraction. Le rapport massique de la phase organique sur la phase aqueuse est de 3,3/1. Les deux phases se rencontrent à contre-courant dans chaque étage.

La phase organique sort par le compartiment mélangeur du troisième étage : elle contient 0,46% d'AA. La phase aqueuse sort par le compartiment mélangeur du premier étage. Elle contient 0,15% d'AA.

Le rendement d'extraction, exprimé par le rapport de l'AA récupéré en phase organique sur l'AA introduit par la phase aqueuse entrante est de 88% à la sortie du troisième étage.

Ce rendement d'extraction, par étage, se décompose ainsi :

- 1 étage : 63% ;
- 2 étages : 85% ;
- 3 étages : 88%.

EXEMPLE 16

Dans un deuxième exemple, les mêmes conditions sont conservées, hormis le fait que l'on ajuste le rapport molaire de soude sur acidité de telle façon que l'AA contenu dans le brut réactionnel (7,02 g) ne soit que partiellement neutralisé par la soude dans la première étape.

Le mélange brut a la composition suivante :

- A2EH : 90% ;
- 2EHOH : 4,5% ;
- AA : 0,95% ;
- Sulfate acide de 2-éthylhexyle : 2,6%.

La phase aqueuse soutirée dans le décanteur situé en tête de la colonne de réaction présente la composition sui-

vante :

- H$_2$O : 98,2% ;
- 2EHOH : 0,09% ;
- AA : 1,7%.

Après neutralisation, l'acidité forte due au sulfate acide de 2-éthylhexyle est totalement neutralisée, et il subsiste une teneur de 0,7% AA dans le brut, soit 4,15 g de AA. On récupère ainsi 59% de l'AA contenu dans le brut initialement.

Ce brut est envoyé dans la batterie des trois mélangeur-décanteurs, comme il a été décrit ci-dessus, en contre-courant d'une phase aqueuse réactionnelle contenant 1,72% d'AA (soit 3,1 g d'AA), avec un rapport massique de la phase organique sur la phase aqueuse de 3,3/1.

On récupère une phase organique sortant par le compartiment mélangeur du troisième étage, qui contient 0,89% d'AA (soit 5,23 g d'AA) ; et une phase aqueuse sortant par le compartiment mélangeur du premier étage, qui contient 1,07% d'AA (soit 2,02 g d'AA).

Le rendement d'extraction, exprimé par le rapport de l'AA récupéré en phase organique sur l'AA introduit par les flux entrants (phase aqueuse réactionnelle et brut neutralisé) est de 72% à la sortie du troisième étage.

## Revendications

1. Procédé de fabrication d'un ester d'acide acrylique par réaction dudit acide et d'un alcool choisi parmi les monoalcools primaires et secondaires aliphatiques ayant un nombre d'atomes de carbone supérieur ou égal à 5, en présence d'un acide comme catalyseur d'estérification et d'un inhibiteur de polymérisation, l'alcool étant capable de former un hétéroazéotrope avec l'eau, l'eau formée par la réaction d'estérification étant entraînée par distillation dans une colonne sous la forme d'un mélange hétéroazéotropique avec l'alcool, lequel mélange, comportant une quantité d'acide acrylique n'ayant pas réagi, est soumis ensuite, après condensation, à une séparation pour donner une phase organique supérieure que l'on recycle en tête de la colonne de distillation et une phase aqueuse inférieure que l'on soutire,
caractérisé par le fait que l'on conduit une extraction de l'acide acrylique contenu dans le mélange hétéroazéotropique condensé adressé à la séparation de phases par un mélange riche en alcool estérifiant, et qu'on renvoie en tête de la colonne de distillation, l'acide acrylique ainsi extrait, en tant que constituant de la phase organique recyclée, avec augmentation du taux de reflux dans la colonne de distillation par rapport au reflux naturel.

2. Procédé selon la revendication 1, dans lequel l'estérification est conduite en continu, caractérisé par le fait que l'on effectue l'opération d'extraction dans une colonne d'extraction (CE) alimentée en tête, par le mélange hétéroazéotropique condensé (M), et en pied, par de l'alcool frais.

3. Procédé selon la revendication 1, dans lequel l'estérification est conduite en continu, caractérisé par le fait que l'on effectue l'opération d'extraction dans un décanteur (D) alimenté, dans la partie supérieure de sa phase organique surnageante (O), par le mélange hétéroazéotropique condensé (M), et, dans la partie inférieure de sa phase aqueuse inférieure (A), par de l'alcool frais, et l'on procède de façon à soutirer à niveau d'interphase constant dans le décanteur (D).

4. Procédé selon la revendication 1, dans lequel l'estérification est conduite en discontinu, caractérisé par le fait que l'on procède comme décrit à la revendication 2.

5. Procédé selon la revendication 1, dans lequel l'estérification est conduite en discontinu, caractérisé par le fait que l'on effectue l'opération d'extraction dans un décanteur (D) dimensionné de façon à pouvoir recevoir au moins la totalité du mélange hétéroazéotropique (M) sans soutirage au fond dudit décanteur (D) ; qu'avant le démarrage de la réaction, on remplit le décanteur (D) d'alcool estérifiant jusqu'au niveau de débordement dans la colonne de distillation (CD) ; qu'on amène le mélange hétéroazéotropique condensé (M), que l'on fait arriver dans la partie supérieure de la phase organique (O) qui se forme dans le décanteur (D), à s'accumuler dans ce dernier pendant tout ou partie de la réaction, le volume de phase organique supérieure (O) déplacé par le volume de mélange hétéroazéotropique accumulé étant reflué (par R) en tête de la colonne de distillation (CD) ; et que, lorsque la réaction est terminée, on soutire la phase aqueuse inférieure (A) et on remplace le volume du mélange hétéroazéotropique éliminé par de l'alcool frais pour une prochaine réaction d'estérification.

6. Procédé selon la revendication 1, dans lequel l'estérification est conduite en discontinu, caractérisé par le fait que l'on procède comme décrit à la revendication 5, la seule différence étant qu'on introduit de l'alcool frais dans le décanteur pendant tout ou partie de la réaction.

**7.** Procédé selon l'une des revendications 1 à 6, caractérisé par le fait qu'on adresse la phase aqueuse issue de l'opération d'extraction à une colonne de distillation (9) en aval, permettant de récupérer les produits organiques légers en tête et de rejeter les eaux épuisées en pied.

**8.** Procédé selon l'une des revendications 1 à 7, caractérisé par le fait que l'on conduit dans la colonne (6) de lavage une extraction des dernières traces d'acide acrylique contenues dans la phase aqueuse (13) issue de l'opération de séparation, par le brut réactionnel (17) débarrassé du catalyseur et de tout ou partie de l'acide acrylique par neutralisation.

**9.** Procédé selon l'une des revendications 1 à 8, caractérisé par le fait que l'on conduit la réaction d'estérification à une température de 80 à 130°C, à pression normale ou sous une pression réduite.

**10.** Procédé selon l'une des revendications 1 à 9, caractérisé par le fait que l'alcool est le 2-éthylhexanol.

## Claims

**1.** Process for the manufacture of an acrylic acid ester by reaction of the said acid and an alcohol chosen from primary and secondary aliphatic monoalcohols having a number of carbon atoms greater than or equal to 5, in the presence of an acid as esterification catalyst and of a polymerization inhibitor, the alcohol being capable of forming a heteroazeotrope with water, the water formed by the esterification reaction being entrained by distillation in a column in the form of a heteroazeotropic mixture with the alcohol, which mixture, containing an amount of unreacted acrylic acid, is then subjected, after condensation, to a separation in order to give an upper organic phase which is recycled to the head of the distillation column and a lower aqueous phase which is withdrawn, characterized in that an extraction of the acrylic acid contained in the condensed heteroazeotropic mixture intended for phase separation is carried out with an esterifying-alcohol-rich mixture, and in that the acrylic acid thus extracted is returned to the head of the distillation column, as a constituent of the recycled organic phase, with an increase in the level of backstreaming in the distillation column relative to the natural backstreaming.

**2.** Process according to Claim 1, in which the esterification is carried out continuously, characterized in that the extraction operation is carried out in an extraction column (EC) supplied, at the head, with the condensed heteroazeotropic mixture (M), and, at the foot, with fresh alcohol.

**3.** Process according to Claim 1, in which the esterification is carried out continuously, characterized in that the extraction operation is carried out in a decanter (D) supplied, in the upper part of its supernatant organic phase (O), with the condensed heteroazeotropic mixture (M), and, in the lower part of its lower aqueous phase (A), with fresh alcohol, and the process is performed so as to withdraw at constant interphase level in the decanter (D).

**4.** Process according to Claim 1, in which the esterification is carried out discontinuously, characterized in that the process is performed as described in Claim 2.

**5.** Process according to Claim 1, in which the esterification is carried out discontinuously, characterized in that the extraction operation is carried out in a decanter (D) of such a size as to be able to receive at least all of the heteroazeotropic mixture (M) without withdrawing at the bottom of the said decanter (D); in that before starting the reaction, the decanter (D) of esterifying alcohol is filled to the overflow level in the distillation column (DC); in that the condensed heteroazeotropic mixture (M), which is led into the upper part of the organic phase (O) which forms in the decanter (D), is accumulated in the latter for all or part of the reaction, the volume of upper organic phase (O) displaced by the volume of accumulated heteroazeotropic mixture being backstreamed (by R) to the head of the distillation column (DC); and in that when the reaction is complete, the lower aqueous phase (A) is withdrawn and the volume of the heteroazeotropic mixture removed is replaced by fresh alcohol for a subsequent esterification reaction.

**6.** Process according to Claim 1, in which the esterification is carried out discontinuously, characterized in that the process is performed as described in Claim 5, the only difference being that fresh alcohol is introduced into the decanter for all or part of the reaction.

**7.** Process according to one of Claims 1 to 6, characterized in that the aqueous phase obtained after the extraction operation is directed to a downstream distillation column (9), which enables the light organic products at the head to be recovered and the waste waters at the foot to be discharged.

8. Process according to one of Claims 1 to 7, characterized in that an extraction of the final traces of acrylic acid contained in the aqueous phase (13) obtained after the separation operation is carried out in the washing column (6), with the reaction crude (17) freed of the catalyst and of all or part of the acrylic acid by neutralization.

9. Process according to one of Claims 1 to 8, characterized in that the esterification reaction is carried out at a temperature from 80 to 130°C, at normal pressure or at reduced pressure.

10. Process according to one of Claims 1 to 9, characterized in that the alcohol is 2-ethylhexanol.

**Patentansprüche**

1. Verfahren zur Herstellung eines Acrylsäureesters durch Reaction der Acrylsäure mit einem aus primären und sekundären, 5 oder mehr Kohlenstoffatome enthaltenden, aliphatischen Monoalkoholen ausgewählten Alkohol in Gegenwart einer Säure als Veresterungskatalysator und eines Polymerisationsinhibitors, wobei der Alkohol mit Wasser ein Heteroazeotrop bilden kann, das bei der Veresterungreaktion gebildete Wasser durch Destillation in einer Kolonne in Form einer heteroazeotropen Mischung mit dem Alkohol weggeschleppt wird und die Mischung, die eine Menge nicht umgesetzter Acrysäure enthält, nach der Kondensation anschließend einer Trennung unterworfen wird, so daß eine obere organische Phase, die am Kopf der Destillationskolonne wieder eingesetzt wird, und eine untere wäßrige Phase, die man entnimmt, erhalten wird,
dadurch gekennzeichnet, daß man die in der kondensierten und der Phasentrennung zugeführten heteroazeotropen Mischung enthaltende Acrylsäure, mittels einer an Veresterungsalkohol reichen Mischung extrahiert und daß man die auf diese Weise extrahierte Acrylsäure als Bestandteil der wiedereingesetzten organischen Phase mit Erhöhung des Rückflußgrades in der Destillationskolonne, bezogen auf den natürlich Rückfluß, wieder dem Kopf der Destillationskolonne zuführt.

2. Verfahren nach Anspruch 1, bei dem die Veresterung kontinuierlich durchgeführt wird, dadurch gekennzeichnet, daß man den Extraktionsverfahrensschritt in einer Extraktionskolonne (CE) durchführt, die am Kopf mit einer kondensierten heteroazeotropen Mischung (M) und am Fuß mit frischem Alkohol gespeist wird.

3. Verfahren nach Anspruch 1, bei dem die Veresterung kontinuierlich durchgeführt wird, dadurch gekennzeichnet, daß man den Extraktionsverfahrensschritt in einer Dekantiervorrichtung (D) durchführt, die im oberen Teil ihrer oben aufschwimmenden organischen Phase (O) mit einer kondensierten heteroazeotropen Mischung (M) und im unteren Teil ihrer unteren wäßrigen Phase (A) mit frischem Alkohol gespeist wird, und daß man derart vorgeht, daß man die Entnahme auf konstanter Höhe zwischen den Phasen in dem Dekanter (D) durchführt.

4. Verfahren nach Anspruch 1, bei dem die Veresterung diskontinuierlich durchgeführt wird, dadurch gekennzeichnet, daß man gemäß Anspruch 2 verfährt.

5. Verfahren nach Anspruch 1, bei dem die Veresterung diskontinuierlich durchgeführt wird, dadurch gekennzeichnet, daß man den Extraktionsverfahrensschritt in einer Dekantiervorrichtung (D) durchführt, die so dimensioniert ist, daß sie mindestens die gesamte heteroazeotrope Mischung (M) ohne Entnahme am Boden des Dekanters (D) aufnehmen kann ; daß der Dekanter (D) vor Reaktionsstart bis zur Überlaufhöhe in der Destillationskolonne (CD) mit Veresterungsalkohol aufgefüllt wird ; daß man die kondensierte heteroazeotrope Mischung (M), die man dem oberen Teil der organischen Phase (O) zuführt, die sich in dem Dekanter (D) bildet, sich während der gesamten oder eines Teils der Reaktion in letzterem ansammeln läßt, wobei das Volumen der oberen organischen Phase (O), die durch das Volumen der angesammelten heteroazeotropen Mischung ersetzt wird, (mittels R) wieder zum Kopf der Destillationskolonne (CD) zurückfließt und daß man, wenn die Reaktion abgeschlossen ist, die untere wäßrige Phase (A) abzieht und man das Volumen der entfernten heteroazeotropen Mischung durch frischen Alkohol für die nächste Veresterungsreaktion ersetzt.

6. Vefahren nach Anspruch 1, bei dem die Veresterung diskontinuierlich durchgeführt wird, dadurch gekennzeichnet, daß man gemäß Anspruch 5 verfährt, jedoch mit dem einzigen Unterschied, daß man den frischen Alkohol während der gesamten oder eines Teils der Reaktion in den Dekanter einbringt.

7. Verfahren nach einem der Ansprüche 1 bis 6, dadurch gekennzeichnet, daß man die wäßrige Phase, die aus dem Extraktionsverfahrensschritt stammt, einer Destillationskolonne (9) stromabwärts zuführt, die es ermöglicht, die leichteren organischen Produkte am Kopf zu isolieren und die verbrauchten Abwässer am Fuß zu verwerfen.

8. Verfahren nach einem der Ansprüche 1 bis 7, dadurch gekennzeichnet, daß man in einer Waschkolonne (6) die letzten Spuren Acrylsäure, die in der aus dem Trennverfahrensschritt stammenden wäßrigen Phase (13) enthalten sind, mit Hilfe einer Reaktionsrohmischung (17) extrahiert, aus der man mittels Neutralisation den Katalysor und die gesamte oder einen Teil der Acrylsäure entfernt hat.

9. Verfahren nach einem der Ansprüche 1 bis 8, dadurch gekennzeichnet, daß man die Veresterungsreaktion bei einer Temperatur von 80 bis 130°C bei Normaldruck oder unter reduziertem Druck durführt.

10. Verfahren nach einem der Ansprüche 1 bis 9, dadurch gekennzeichnet, daß der Alkohol 2-Ethylhexanol ist.

EP 0 687 664 B1

FIGURE 1 (Etat antérieur de la technique)

FIGURE 2

FIGURE 3

FIGURE 4

Alcool frais

Figure 5

EP 0 687 664 B1